# EUROPÄISCHE PATENTANMELDUNG

(11) **EP 1 574 162 A2**
(43) Veröffentlichungstag der Anmeldung: **14.09.2005**
(21) Anmeldenummer: 05003431.3
(22) Anmeldetag: 17.02.2005
(51) Int. Cl.: A61B 1/267, A61B 1/04

(54) **Bildaufnahmevorrichtung mit mehreren Bildaufnahmemodi**

(30) Priorität: 08.03.2004 DE 102004011147
(71) Anmelder: Fraunhofer-Gesellschaft zur Förderung der angewandten Forschung e.V., 80686 München (DE)
(72) Erfinder: Wittenberg, Thomas, 91054 Erlangen (DE); Heppner, Wolfgang, 90473 Nürnberg (DE); Gick, Stephan, 96215 Lichtenfels (DE); Völlinger, Hubert, 76437 Rastatt (DE); Osterland, Ulrich, 13357 Berlin (DE); Belikan, Thomas, 75015 Bretten (DE); Dolt, Martin, 75438 Knittlingen (DE); Bloss, Hans, 90562 Heroldsberg (DE); Schmidt, Robert, 91058 Erlangen (DE)
(74) Vertreter: Schoppe, Fritz

(57) **Zusammenfassung**

Eine effektivere Kehlkopf- bzw. Stimmlippenuntersuchung wird ermöglicht, wenn eine Bildaufnahmevorrichtung (10), vorzugsweise ein Farbbildaufnahmevorrichtung, mit drei Modi zur Verfügung gestellt wird, einem flächenhaft aufnehmenden Aufnahmemodus mit einer niedrigen Aufnahmefrequenz bzw. Bildwiederholfrequenz aber dafür einer großen Auflösung, einem weiteren flächenhaft aufnehmenden Aufnahmemodus mit einer höheren Bildwiederholfrequenz aber dafür einer geringeren Auflösung und einem eindimensionalen, beispielsweise Zeilen- Aufnahmemodus mit einer ebenfalls höheren Bildwiederholfrequenz. Durch die Umschaltbarkeit der Bildaufnahmevorrichtung zwischen diesen Modi ist es dem untersuchenden Arzt möglich, sich zunächst mittels des langsameren Bildaufnahmemodus zunächst einen Überblick zu verschaffen, wobei dies auch in Farbe geschehen kann, da durch die langsamere Aufnahmefrequenz dieser Modus nicht den Beleuchtungsproblemen wie die Hochgeschwindigkeitskamera unterliegt.

## Beschreibung

Die vorliegende Erfindung bezieht sich auf Bildaufnahmevorrichtungen im allgemeinen und in Ausführungsbeispielen auf Kameras, die zu Untersuchungen im Kehlkopf und besonders für die Beobachtung von Schwingungen der Stimmlippen oder der Ersatzstimmlippen geeignet sind.

In Deutschland suchen jährlich ca. 30.000 neue Patienten wegen Heiserkeit bzw. Beschwerden ihrer Stimme einen Facharzt für Phoniatrie und Pädaudiologie auf. Von diesen Neuvorstellungen sind mehr als die Hälfte beruflich auf ihre Stimme angewiesen. Insbesondere gehören Lehrer, Politiker, Vertreter, Pfarrer, Sänger, Schauspieler und Moderatoren zu dieser Gruppe. Besonders bei Pädagogen sind Beeinträchtigungen der Stimme häufig anzutreffen und aus arbeitsmedizinischer Sicht überdurchschnittlich signifikant. Bei einer Untersuchung 1970 wurden bei 7 bis 8% unter einer Gruppe von untersuchten Lehrern Stimmstörungen festgestellt. Neuere Untersuchungen unter Lehramtsstudenten an der Universität Essen ergaben, dass fast 30% von ihnen an Störungen der Stimme litten. Auch bei einer Befragung 1994 unter Lehrerinnen und Lehrern gaben 76% an, gelegentlich bis oft stimmlich beeinträchtigt zu sein. Die Mehrheit dieses Personenkreises konsultiert nach eigener Aussage aus diesem Grund auch einen Arzt und ist zeitweise arbeitsunfähig.

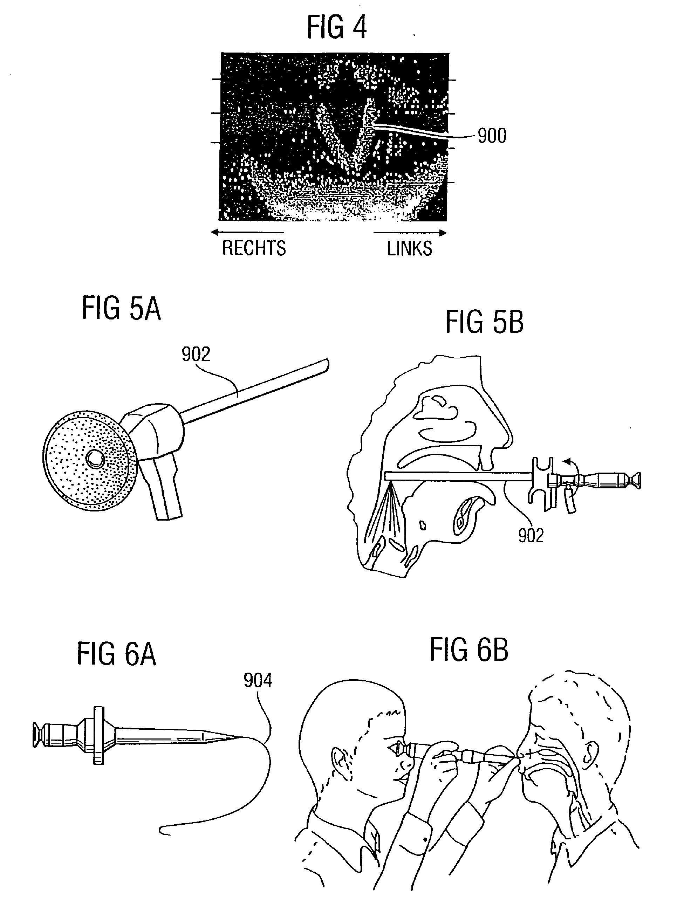

Eine wesentliche Komponente der dann folgenden fachärztlichen Untersuchung ist die visuelle Inspektion des Organs der Stimmentstehung, d.h. des Kehlkopfes. Dabei betrachtet der Phoniater den im Hals gelegenen Kehlkopf mit den Stimmbändern mittels eines Kehlkopfspiegels oder mit einem Endoskop. Fig. 4 zeigt ein Endoskopiebild eines gesunden Kehlkopfes in Atemstellung. Die unterhalb des Bildes abgegebene "Orientierung" bezieht sich auf die Sicht des Patienten. Durch das Endoskop blickt man von oben auf den Kehlkopf (Larynx). Das ovale, dunkle Areal in der Bildmitte ist die weit geöffnete Stimmritze (Glottis), rechts und links begrenzt von den im Bild an ihrer V-Form zu erkennenden Stimmlippen 900, im Volksmund Stimmbänder genannt. Die Strukturen am Rand des Bildes, wie z.B. Taschenfalten, Stellknorpel beidseitig und Kehldeckel vorne, sind für das primäre Stimmsignal weniger wichtig, jedoch für die präphonatorische Motorik von Bedeutung.

Im Ruhe- oder Atemzustand lassen sich morphologische Veränderungen im Kehlkopfbereich, wie beispielsweise die Neubildung von gut- oder bösartigem Gewebe, wie z.B. Polypen, Karzinomen, Zysten und Granulomen, überlastungsbedingte Verdickungen der Stimmlippen (Schreiknötchen) oder entzündliche Veränderungen als Ursache für Stimmerkrankungen erkennen. Sieht der Arzt keine organischen Veränderungen, so führt er die heisere Stimme auf "unregelmäßige" Schwingungen der Stimmlippen zurück und stellt die Diagnose "funktionelle Störung".

Während sich statisch-morphologische Veränderungen direkt, d.h. durch Betrachten mit einem Endoskop diagnostizieren lassen, sind zur Untersuchung der Stimmlippenschwingungen technische Beobachtungshilfen, wie die Video-Stroboskopie oder der Einsatz von Hochgeschwindigkeitskameras erforderlich, da die visuelle Wahrnehmung des Untersuchers die Stimmlippenschwingungen mit 100-400 Schwingungen pro Sekunde nicht mehr verfolgen kann. Neben der rein statischen Untersuchung des Kehlkopfes unter Zuhilfenahme eines starren oder flexiblen Endoskops wird heute weltweit ein stroboskopisches Verfahren eingesetzt. Alternativen speziell für die Untersuchung von Stimmlippenschwingungen bieten die in den vergangenen 10 Jahren entwickelten Verfahren der Videokymographie sowie die Verwendung von Hochgeschwindigkeitskameras, wobei all diese Verfahren im folgenden näher erörtert werden.

Die traditionelle Untersuchung des Kehlkopfes geschieht seit dem 19. Jahrhundert mit einem Kehlkopfspiegel. Dieser wird zunehmend durch starre Lupenendoskope (Staboptiken), oder flexible Endoskope (Fiberskope) abgelöst, an die sich auch Foto- oder Videokameras adaptieren lassen.

Wegen der einfachen Handhabung bei gleichzeitiger optimaler Bildqualität wird der Kehlkopf heute meist mit einem Lupenlaryngoskop untersucht, wie es beispielsweise in Fig. 5a gezeigt ist.

Diese Endoskope besitzen eine starre Staboptik mit einem Umlenkprisma mit einem Umlenkwinkel von 90° oder 70° am Lichteintritt (nicht gezeigt) und zwei Vergrößerungsbereichen. Das starre Rohr 902 des Endoskops besitzt einen Durchmesser von ca. 9 mm und nimmt neben dem starren Glaskern des optischen Kanals auch Kanäle für eine oder mehrere Lichtleitungen auf.

Das Lupenendoskop wird bei der Untersuchung durch den Mund bis zur Rachenhinterwand geschoben, wie es in Fig. 5b gezeigt ist. Dabei muss die Zunge des Patienten herausgezogen werden, um den Blick auf den Kehlkopf freizuhalten. Bei überhängendem Kehldeckel können die Stimmlippen nur bei der Phonation von Vokalen wie /i/ und /ä/ beobachtet werden.

Eine alternative Möglichkeit zur Untersuchung des Kehlkopfes bietet eine flexible Optik, das sogenannte Fiberskop. Ein Fiberskop ist exemplarisch in Fig. 6a gezeigt. Es umfasst ein ca. 3-5 mm starkes Glasfaserbündel 904 für die Lichtleitung und die optische Übertragung. Flexible Endoskope besitzen schlechtere optische Eigenschaften bezüglich ihres Abbildungs- und Vergrößerungsvermögens als starre Endoskope.

Dementsprechend ist der sichtbare Bildausschnitt viel kleiner bzw. der Vergrößerungsfaktor wesentlich geringer als bei Laryngoskopen.

Das flexible Endoskop wird nach einer Oberflächenanästhesie der Schleimhaut durch Nasen-, Nasenrachenraum und Mundrachenraum bis ungefähr 2 cm oberhalb des Kehlkopfes geschoben, wie es in Fig. 6b gezeigt ist. Die ca. 2 cm lange starre Spitze des Glasfaserbündels lässt sich dort nach dem Einführen um ca. 60 Grad auf- und abbewegen.

Das flexible Endoskop beeinträchtigt Phonation und Artikulation nicht wesentlich. Damit können Stimmlippenschwingungen auch während des Sprechens beobachtet werden. Nachteile von flexiblen Endoskopen sind die geringere Ausleuchtung und die niedrigere räumliche Bildauflösung.

Konventionelle optische Aufnahmesysteme wie Film- und Videokameras besitzen in der Regel ein begrenztes zeitliches Auflösungsvermögen mit maximalen Aufnahmefrequenzen von 25 (PAL) oder 30 (NTSC) Vollbildern bzw. 50 (PAL) oder 60 (NTSC) Halbbildern pro Sekunde. Diese beschränkte zeitliche Auflösungsfähigkeit ist motiviert durch die visuelle Wahrnehmung des Menschen, die eine obere Grenzfrequenz von 25 Hz besitzt. Diese Aufnahmeraten sind ausreichend, um biomechanische Bewegungen mit periodischen Frequenzen bis zu ca. 15 Hz, wie beispielsweise das Pulsieren des gesunden menschlichen Herzens oder die Fortbewegung durch Gehen, genügend genau abzutasten.

In der Medizin gibt es jedoch einige Anwendungen mit Bewegungen, deren Grundfrequenzen oberhalb dieser 25 Hz liegen, und die bei der Video-Aufnahme eine wesentlich höhere zeitliche Auflösung erfordern. Beispiele dafür sind die Gang-, Lauf- und Sprunganalyse in der Orthopädie und der Sportmedizin, die Öffnung und Verschlussbewegung von Herzklappen oder Schwingungen von Stimmlippen während des Sprechens oder Singens. Die Grundfrequenz dieser Stimmlippenschwingungen liegt während des normalen Sprechens im Bereich zwischen 100 und 400 Hz.

Um für eine beliebige periodische Bewegung mit einer maximalen Objektfrequenz ƒ₀ das passende zeitliche Auflösungsvermögen F eines Aufnahmesystems zu finden, kann das sog. Abtasttheorem von Claude Shannon herangezogen werden. Dieses besagt, dass ein ausreichend bandbegrenztes, kontinuierliches Signal ƒ(t) mit Grenzfrequenz ƒ₀ mit den Abtastwerten s₁, s₂, ..., s genau dann eindeutig rekonstruierbar ist, wenn es mit einer Abtastfrequenz F=1/Δt>2 ƒ abgetastet wurde.

Die Schwingungsfrequenz ƒ der menschlichen Stimmlippen liegt mit ca. 100-400 Hz jedoch wesentlich oberhalb der Aufnahmefrequenzen von konventionellen Kameras und ist somit nach dem Abtasttheorem theoretisch weder direkt noch indirekt mit derartigen konventionellen Aufnahmetechniken zu untersuchen.

Für die klinische Differentialdiagnose von funktionellen Stimmstörungen ist zudem die Kurzzeitvariabilität in der Grundfrequenz ("Jitter") und Amplitude ("Shimmer") von Interesse. Aus diesem Grund wird an eine adäquate klinische Aufnahmetechnik nicht nur die Shannon'sche Minimalforderung von F>2ƒ, also mindestens der zweifachen Grundfrequenz ƒ des abzutastenden Signals gestellt, sondern es wird zudem eine Überabtastung in der Größenordnung von einem Faktor 5 - 10 benötigt.

Um die menschlichen Stimmlippenschwingungen, unter den angeführten technischen Randbedingungen aufzunehmen, zu archivieren und gegebenenfalls automatisch zu analysieren, haben sich im Bereich der Phoniatrie mehrere unterschiedliche Aufnahmetechniken etabliert.

Für die Untersuchung von Stimmlippenschwingungen am weitesten verbreitet ist ein stroboskopisches Verfahren, das vom Stimmschall getriggert wird. In der Regel werden die stroboskopischen Bilder der Stimmlippen während der Stimmgebung, der "Phonation", mit einer Videokamera aufgenommen, auf Videoband mitgeschnitten und später durch den Arzt qualitativ beschrieben. Zwei weitere - jüngere - Verfahren, die sich derzeit beide in einer Phase der klinischen Evaluierung befinden, sind die sog. Video-Kymographie sowie die Aufnahme von Stimmlippenschwingungen mit digitalen Hochgeschwindigkeitskameras.

Die *Stroboskopie* ist die zur Zeit gebräuchlichste Methode zur klinischen Untersuchung der Stimmlippenbewegungen. Über ein Kehlkopfmikrophon wird das akustische Signal einer Phonation aufgenommen. Mit der daraus automatisch bestimmten Grundfrequenz wird eine phasengleiche Impulsbelichtung durch eine Stroboskopieblitzlampe erzeugt. Dadurch erscheint dem Beobachter die Schwingung der Stimmlippen als stehendes Bild. Durch eine minimale Phasenverschiebung zwischen der Grundfrequenz des akustischen Signals und der Blitzbeleuchtung des Kehlkopfs nimmt der Beobachter eine "virtuelle", d.h. scheinbar langsame Bewegung der Stimmlippen wahr.

Die Stroboskopie wird beispielsweise in Schulz-Coulon (1980): Die Diagnostik der gestörten Stimmfunktion, in Archiv für Ohren-, Nasen- und Kehlkopfheilkunde, Bd. 227, S. 1-169, Springer Verlag, Berlin, S. 53, oder in Arnold usw.: Clinical Examination of Voice, Springer Verlag, Wien, S. 49, beschrieben.

Die Stroboskopie kann bei gesunden und bei wenig gestörten Stimmen mit quasiperiodischem und symmetrischem Schwingungsverhalten der Stimmlippen brauchbare Ergebnisse liefern. Ihr Einsatz ist jedoch bei funktionell gestörten Stimmen aus ärztlicher Sicht umstritten und aus technischer Sicht aufgrund des Abtasttheorems unmöglich. Bei einer ungeeigneten Blitzauslösung der Stroboskopie täuschen sog. "Aliasingeffekte" Schwingungsverläufe vor, die real nicht bestehen. Darüber hinaus sind insbesondere Perioden- und Amplitudenschwankungen, aperiodisches Schwingungsverhalten und Einschwingvorgänge der Stimmlippen mit der Stroboskopie nicht erfassbar. Zudem können weder Stimmeinsätze noch extrem heisere Stimmen mit der Stroboskopie registriert werden, da sich beide durch ein asynchrones und aperiodisches Schwingungsverhalten der Stimmlippen auszeichnen.

Seit 1978 ergänzt der Einsatz von Videokameras die Stroboskopie als Diagnose- und Archivierungsmethode. Aufgrund der technischen Restriktionen von Videokameras werden die Schwingungen der Stimmlippen jedoch nur mit maximal 30 Bildern bzw. 60 Halbbildern pro Sekunde aufgenommen und somit die Stimmlippenschwingungen unterabgetastet. Die Archivierung der Stroboskopieaufnahmen auf Videobändern und deren nachträgliche Digitalisierung mit hochauflösenden Video-Framegrabbern ermöglicht eine subjektive Beurteilung der Aufnahmen. Eine quantitative Auswertung von digitalisierten Larynx- und Videostroboskopieaufnahmen ist jedoch wegen der zeitlichen Unterabtastung der Bewegung nur für die Textur-und Farbkomponenten sinnvoll.

Zusammenfassend sind die Vor- und Nachteile der Video-Stroboskopie wie folgt:

### Vorteile:

- Gute Bildqualität, hoch auflösend (ca. 700 x 500 Bildpunkte, Videonorm)
- Farbige Aufnahmen
- Erfassung des Schwingungsvorgangs simultan an der ganzen Glottis

### Nachteile

- Keine kontinuierliche Bildfolge (zeitliche Unterabtastung, Verletzung des Abtasttheorems)
- Naturgetreue (aber virtuelle) Abbildung der Schwingung nur bei streng periodischen Schwingungen
- Nur subjektive Beurteilung der Bewegung möglich
- Quantitative Auswertung erst nach aufwändiger Digitalisierung der Aufnahmen möglich
- Bewegungsartefakte und Bildunschärfe durch sog. Interlacing-Effekte (d.h. räumliche Verschachtelung zweier zeitlich nacheinander aufgenommenen Halbbilder)

Als "*Kymographie"* bezeichnet der Mediziner eine Abbildung von eindimensionaler Bewegung in einem zweidimensionalen Bild. Im Falle der Kehlkopfkymographie wird eine einzelne Zeile des Bildausschnittes zu verschiedenen Zeitpunkten betrachtet und untersucht. Von den in der Vergangenheit vorgestellten Varianten dieses Ansatzes konnte sich nur die sogenannte "Videokymographie" etablieren und wird derzeit in einigen Forschungszentren als Ergänzung zur Video-Stroboskopie verwendet.

Durch eine Kooperation der Universität in Groningen mit der holländischen Firma "Lambert Instruments BV" entstand 1976 ein Hochgeschwindigkeits-Video-Kymographiesystem. Dies ist beispielsweise in Svec, Schutte: Videokymographie - High-Spees Line Scanning of Vocal Fold Vibration. Journal of Voice 10 (2), S. 201-205, in Schutte, Svec, Sram: Videokymography - A Modern Imaging System for Analyzing Regular and Irregular Vibrations, in: Aktuelle phoniatrischpädaudiologische Aspeke 1996, Göttingen, S. 272-274 und in Schutte, Svec, Sram: Videokymographie, Imaging and Quantification of Regular and Irregular Vocal Fold Vibrations, in: Proceedings of the XVI World Congress of Otorhinolaryngology Head and Neck Surgery, S. 1739-1742, beschrieben.

Dieses System basiert auf einer Schwarz-Weiß CCD-Videokamera, die anstelle von Vollbildern pro Bild nur eine einzelne Bildzeile (Zeilenkamerasystem) aufzeichnet. Durch die Reduktion des Vollbildes mit 625 bzw. mit 525 Zeilen auf eine einzelne Zeile können Abtastraten bis zu 7812,5 Hz erzielt werden. Diese Zeilenrate reicht aus, um Stimmlippenschwingungen genügend hoch abzutasten, allerdings nur mit einer einzelnen Zeile pro Bild. Alle sukzessiv aufgenommenen Zeilen werden auf einem Videoband aufgezeichnet und können anschließend über einen kommerziellen Videomonitor derart abgespielt werden, dass die vertikale Achse eines Bilds durch die Zeitachse der Aufnahme ersetzt wird.

Die Archivierung der Kymographieaufnahmen wird auf Videobändern durchgeführt, eine Digitalisierung ist möglich.

Die Vor- und Nachteile der Video-Kymographie lassen sich wie folgt zusammenfassen:

### Vorteile:

- Relativ gute Bildqualität, hoch auflösend (ca. 500 Bildpunkte pro Bildzeile, Videonorm)
- Sehr hohe Aufnahmerate (ca. 8000 Hz), d.h. das Abtasttheorem (>2f) inklusive einem Überabtastungsfaktor der Größenordnung 5 (>5) ist für Schwingungsfrequenzen bis ca. 800 Hz (2·5·800 Hz = 8000 Hz) erfüllt.

### Nachteile:

- Schwarz-Weiß Aufnahmen
- Erfassung des Schwingungsvorgangs nur an einer Stelle der Glottis
- Quantitative Auswertung erst nach aufwändiger Digitalisierung der Aufnahmen möglich.
- Erlaubt nur eine subjektive Beurteilung
- Bewegungsartefakte und Bildunschärfe durch sogenannte Interlacing-Effekte (d.h. räumliche Verschachtelung zweier zeitlich nacheinander aufgenommenen Halbbilder)

*Hochgeschwindigkeitsaufnahmen* der schwingenden Stimmlippen wurden erstmalig 1938 gemacht, wie z.B. in High-Speed Motion Pictures of the Vocal Cords, Bureau of Publication, Bell Telephone Labs, New York, 1937 und in Farnsworth, High-Speed Motion Pictures of the Human Vocal Cords, Bell Telephone Records, Band 18, S. 203-208 beschrieben. Dabei kam ein analoges Hochgeschwindigkeitskamerasystem zum Einsatz, bei dem die Filme nach der Belichtung zunächst entwickelt wurden, bevor sie mit Hilfe eines Projektors betrachtet werden konnten. Diese Technologie wurde bis in die späten 60er Jahre des 20ten Jahrhunderts experimentell verwendet, wurde dann aber aufgrund der immensen Nachteile dieses Aufnahmeverfahrens von der oben beschriebenen Stroboskopie abgelöst.

Nunmehr befinden sich *digitale Hochgeschwindigkeitskameras* unterschiedlicher Bauart weltweit im klinischen Erprobungsstadium und bieten damit eine Ergänzung zur stroboskopischen Untersuchung und eine Alternative zur Videokymographie. Für die Anwendung solcher Kameras auf die Aufnahmen von Stimmlippenschwingungen zur Diagnose und Therapieverlaufskontrolle sind beispielsweise Aufnahmen mit 4000 Bildern pro Sekunde und einer räumlichen Auflösung von 256 x 256 Bildpunkten machbar. Die Aufnahmedauer wird dabei durch die Größe des Kameraspeichers begrenzt. D.h. bei einer räumlichen Auflösung von 256 x 256 Bildpunkten und einem Kompressionsfaktor der Größenordnung 2 wird für eine Aufnahmedauer von 2 Sekunden ein Arbeitsspeicher mit 256 Megabyte benötigt. Dieser Arbeitsspeicher lässt sich erweitern, so dass auch längere Aufnahmen möglich sind, was aber in der klinischen Routine nicht benötigt wird, da die klinisch interessanten und diagnostisch relevanten Aktionen der Stimmlippen in der Regel in Größenordnungen von 300-500 Millisekunden liegen.

Nachstehend werden die Vor- und Nachteile von digitalen Hochgeschwindigkeitskameras zusammengefasst:

### Vorteile:

- Hohe Aufnahmeraten (ca. 4000 Hz), d.h. das Abtasttheorem (>2f) inklusive einem Überabtastfaktor der Größenordnung 5 (>5) ist für Schwingfrequenzen bis ca. 400 Hz (2·5·400 Hz = 4000 Hz) erfüllt.
- Erfassung des Schwingungsvorgangs simultan an der ganzen Glottis
- Digitale Kymogramme lassen sich nachträglich an einer beliebigen Stelle der Glottis aus gespeicherten Aufnahmen berechnen
- Quantitative Auswertung direkt nach der Abspeicherung auf einen Rechner möglich.

### Nachteile:

- Mittlere - aber akzeptable - Bildqualität, d.h. ca. 256 x 256 Bildpunkte.
- Aktuell für den Phoniatrischen Bereich nur als Schwarz-Weiß-System verfügbar.
- Farb-Hochgeschwindigkeitskameras sind technisch realisiert (siehe z.B. Crashtest-Systeme) und von den Kliniken gewünscht, aber für den Bereich der Phoniatrie bisher noch nicht technisch umgesetzt worden. Da solche Farbsysteme in der Regel eine 2-3 mal hellere Beleuchtung benötigen, die entsprechend den Rachen erhitzt, sind diese für den genannten medizinischen Einsatz (a) zu dunkel (Normale Lichtquelle, wenig Hitze, hohe Aufnahmerate), (b) zu langsam (normale Lichtquelle, wenig Hitze, langsame Aufnahmerate) oder (c) zu heiß (Lichtquelle mit hoher Leistung, viel Hitze, aber hohe Aufnahmerate).

Aus der vorhergehenden Beschreibung wird deutlich, dass Medizinern bis heute kein System zur Verfügung steht, das es ermöglicht, ohne irgendwelche Nachteile visuelle Untersuchungen am Kehlkopf durchzuführen. Selbst wenn ein Arzt alle oben beschriebenen Systeme, d.h. Laryngoskopie, Stroboskopie, Kymographie und digitale Hochgeschwindigkeitskamera, zur Verfügung stehen hätte, müsste er mit all diesen Nachteilen leben und zudem mit einer umständlichen Handhabung dreier Geräte bzw. Systeme. Es besteht folglich ein Bedarf nach einem System, das eine effektivere bildgebende Kehlkopfuntersuchung ermöglicht.

Die Aufgabe der vorliegenden Erfindung besteht folglich darin, eine Bildaufnahmevorrichtung zu schaffen, die eine effektivere Kehlkopf- bzw. Stimmlippenuntersuchung ermöglicht.

Diese Aufgabe wird durch eine Bildaufnahmevorrichtung gemäß Anspruch 1 gelöst.

Die Erkenntnis der vorliegenden Erfindung besteht darin, dass eine effektivere Kehlkopf- bzw. Stimmlippenuntersuchung ermöglicht wird, wenn eine Bildaufnahmevorrichtung, vorzugsweise eine Farbbildaufnahmevorrichtung bzw. Farbkamera, mit zumindest zwei von drei Modi zur Verfügung gestellt wird, und zwar (a) mit einem flächenhaft aufnehmenden Aufnahmemodus mit einer niedrigen Aufnahmefrequenz bzw. Bildwiederholfrequenz aber dafür mit einer großen räumlichen Bildauflösung, (b) einem weiteren flächenhaft aufnehmenden Aufnahmemodus mit einer höheren Bildwiederholfrequenz aber dafür einer geringeren räumlichen Bildauflösung, und (c) einem eindimensionalen, beispielsweise Zeilen-Aufnahmemodus mit einer ebenfalls höheren Bildwiederholfrequenz.

Bei Umschaltbarkeit der Bildaufnahmevorrichtung zwischen den drei Modi ist es dem untersuchenden Arzt möglich, sich zunächst mittels des langsameren Bildaufnahmemodus einen Überblick zu verschaffen (Laryngoskopie). Danach kann der Arzt mit dem ebenfalls flächenhaft aufnehmenden aber schnelleren Aufnahmemodus eine erste Auswertung einer Zeitlupe der Stimmlippenbewegung durchführen. Aufgrund dieser Aufnahme kann dann der Arzt je nach Notwendigkeit dann auch eine Kymographie mittels des eindimensional aufnehmenden Bildaufnahmemodus durchführen, wobei er aufgrund der Möglichkeit des Umschaltens zwischen den Modi dabei die Abtastlinie derart geeignet zu den Stimmbändern ausrichten kann, dass die Abtastlinie an einem geeigneten Ort relativ zu den Stimmbändern angeordnet ist. Das Zusammenspiel aller drei Modi und die Umschaltbarkeit ermöglicht folglich eine in allen Belangen problemlose Kehlkopf- und Stimmlippenuntersuchung. Bei fehlendem dritten Aufnahmemodus kann ein Kymogramm alternativ aus der schnelleren Aufnahme im zweiten Aufnahmemodus erzeugt werden, wenn eine Zeile aus dem zweidimensionalen Feld herausgegriffen wird.

Bevorzugte Ausführungsbeispiele der vorliegenden Erfindung werden nachfolgend bezugnehmend auf die beiliegenden Zeichnungen näher erläutert. Es zeigen:
- Fig. 1: ein Blockschaltbild eines Laryngoskopiesystems gemäß einem Ausführungsbeispiel der vorliegenden Erfindung;
- Fig. 2: eine schematische Zeichnung der Kamera in dem System von Fig. 1 gemäß einem Ausführungsbeispiel der vorliegenden Erfindung;
- Fig. 3: eine schematische Zeichnung des Pixelarrays der Kamera von Fig. 2 zur Veranschaulichung der unterschiedlichen Bereiche von Bildpunkten, die bei den einzelnen Bildaufnahmemodi der Kamera verwendet werden, gemäß einem Ausführungsbeispiel der vorliegenden Erfindung;
- Fig. 4: ein Endoskopiebild eines gesunden Kehlkopfes in Atemstellung;
- Fig. 5a: ein Raumbild eines starren Endoskops;
- Fig. 5b: eine schematische Zeichnung zur Veranschaulichung des Einsatzes des starren Endoskops von Fig. 5a zur Untersuchung des Kehlkopfes;
- Fig. 6a: ein Bild eines Fiberskops;
- Fig. 6b: eine schematische Zeichnung zur Veranschaulichung des Einsatzes des Fiberskops von Fig. 6a zur Untersuchung des Kehlkopfes.

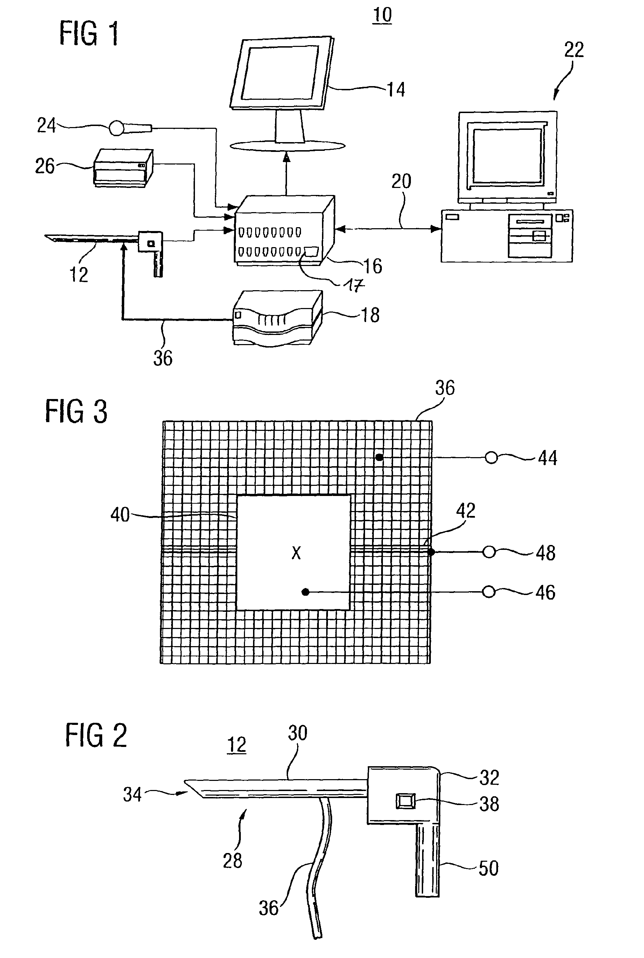

Fig. 1 zeigt ein Gerät zur Untersuchung des Kehlkopfes bzw. der Stimmlippen gemäß einem Ausführungsbeispiel der vorliegenden Erfindung. Das System, das allgemein mit 10 angezeigt ist, umfasst eine Kamera 12, deren Aufbau im folgenden bezugnehmend auf Fig. 2 näher erläutert wird, einen Video-Monitor 14 mit beispielsweise einem Standard PAL- oder NTSC-Eingang, ein Steuergerät 16 mit einem digitalen Bildspeicher 17 und eine Lichtquelle 18. Das Steuergerät 16 ist ferner über eine Netzwerk-, wie z.B. eine Ethernet-, verbindung 20 mit einem Computer 22 verbindbar. Ferner sind an das Steuergerät weitere externe Geräte anschließbar, wie z.B. ein Mikrophon 24 und ein EGG-Gerät 26, wobei jedoch alternativ oder zusätzlich auch andere Geräte angeschlossen sein können.

Fig. 2 veranschaulicht die Kamera 12 detaillierter. Gemäß dem Ausführungsbeispiel von Fig. 2 ist die Kamera 12 mit einem starren Endoskopaufsatz 28 mit einer Staboptik 30 vorgesehen, das wie bezugnehmend auf Fig. 5b beschrieben, dazu vorgesehen ist, durch den geöffneten Mund in den Rachenraum des Patienten eingeschoben zu werden. An einer Ankoppelungsseite des Endoskops 28 ist dasselbe an einen Kamerakopf 32 ankoppelbar. An dem anderen, distalen Ende des Endoskops 28 befindet sich die optische Öffnung 34, über das das vom Kehlkopf reflektierte Licht zum Kamerakopf 32 gelangt. Die optische Öffnung 34 ist ferner dazu da, ein über einen an das Endoskop 28 ankoppelbaren Lichtleiter 36 von der Lichtquelle 18 zugeführtes Licht zur Beleuchtung des Kehlkopfes bzw. der Stimmlippen zu letztgenannten hindurchtreten zu lassen. Die Optik des Endoskops 28 bildet den zu untersuchenden Kehlkopf bzw. die Stimmlippen auf einen lichtempfindlichen Bereich, bzw. eine lichtempfindliche Fläche des Kamerakopfes 32 ab, die durch ein Pixelarray gebildet wird, das beispielsweise als ein CMOS-Chip implementiert ist.

Fig. 3 zeigt exemplarisch das Pixelarray 36 der Kamera 32. Es umfasst exemplarisch 512 x 386 Bildpunkte. In Fig. 3 ist zur Übersichtlichkeit das Pixelarray 36 lediglich als die Fläche, die die Bildpunkte des Pixelarrays einnehmen, und nicht als die Menge der Bildpunkte bzw. Pixel dargestellt, die zeilen- und spaltenmäßig regelmäßig angeordnet sind. In Fig. 3 ist durch ein kleines Kreuz "x" derjenige Punkt auf der Fläche des Pixelarrays 36 angezeigt, in der eine optische Achse der Kamera 12 bzw. der abbildenden Optik 28 das Pixelarray schneidet.

Die Kamera 12 ist durch ein Bedienelement 38, wie z.B. eine Bedientaste, ein Knopf, ein Drehschalter, ein Kippschalter oder dergleichen, zwischen drei Betriebs- bzw. Bildaufnahmemodi umschaltbar. In einem ersten Bildaufnahmemodus zeichnet die Kamera Bilder mit einer langsamen Bildwiederholfrequenz bzw. Aufnahmefrequenz von beispielsweise 20-1000Hz oder von nur 20-100 Hz, vorzugsweise 25Hz oder 30 Hz, auf, und zwar mit einer Auflösung, die alle 512 x 386 Bildpunkte des Pixelarrays 36 umfasst. In einem zweiten Bildaufnahmemodus zeichnet die Kamera Bilder mit einer höheren Geschwindigkeit auf, nämlich beispielsweise mit einer Geschwindigkeit von 1000 bis 4000 Hz, wobei jedoch zur Aufzeichnung nur die Bildpunkte innerhalb eines vorzugsweise rechteckigen Teilbereiches 40 von etwa 200 x 200 bis 500 x 500 und vorzugsweise 256 x 256 Bildpunkten verwendet wird, wobei der Teilbereich 40 im wesentlichen zentrisch also mittig zur optischen Achse angeordnet ist. In einem dritten Bildaufnahmemodus zeichnet die Kamera 12 Bilder mit einer noch höheren Bildwiederholfrequenz von größer als 4000 Hz, wie z.B. 8000 Hz, auf, verwendet dafür allerdings lediglich eine Bildpunktlinie, wie z.B. eine Zeile von 512 Bildpunkten des Pixelarrays 36, wobei die Bildzeile mit 42 angezeigt ist und vorzugsweise durch die optische Achse x verläuft. Das Pixelarray 36 umfasst folglich einen Ausgang 44 zum Ausgeben der Bilddaten in einem ersten Aufnahmemodus, der für die Laryngoskopie, also die normale Untersuchung des Kehlkopfes vorgesehen ist, einen Ausgang 46 zum Ausgeben der Bilddaten in den zweiten Bildaufnahmemodus, der für Hochgeschwindigkeitsaufnahmen vorgesehen ist, und einen Ausgang zum Ausgeben der Bilddaten in den dritten Bildaufnahmemodus, der für die digitale Kymographie vorgesehen ist. Die Ausgänge 44-48 müssen nicht physisch getrennte Ausgänge sein, sondern können auch durch einen Ausgang gebildet sein. In dem Fall der Implementierung durch einen CMOS-Sensor-Array wird die Realisierung der verschiedenen Auslesebereiche 36, 40 bzw. 42 durch die einzelne Adressierbarkeit der Bildpunkt- bzw. Sensorelemente erzielt. Hinsichtlich der Größe des Pixelarrays 36 ist auch eine andere Größe als 512 - x 386 Bildpunkte, wie beispielsweise 1000 x 1000 möglich. Ferner wäre es möglich auch als den Pixelbereich für den ersten Bildaufnahmemodus nur einen Teilbereich des gesamten Pixelarrays zu verwenden.

Nach dem im vorhergehenden der Aufbau des Systems 10 und auch der Kamera 12 genauer beschrieben worden ist, wird im folgenden dessen Funktionsweise sowie die Funktionsweise der Kamera 12 innerhalb des Systems 10 beschrieben.

Wenn das System 10 eingeschaltet wird, befindet sich beispielsweise die Kamera 12 vorgabemäßig zunächst im langsamen Vollbildmodus. Wenn das Endoskop 28 noch nicht auf den Kamerakopf 32 gesteckt ist, so kann dies nachgeholt werden. Durch einen speziellen Adapter (nicht gezeigte Fig. 2) an der Kamera 12, die beispielsweise eine Schnapp- oder Stecktechnik verwendet, können für die Aufnahme Endoskope unterschiedlicher Bauart verwendet werden, obwohl in Fig. 2 exemplarisch nur ein starres Endoskop gezeigt ist. So können auch Faserendoskope verwendet werden, und alternativ auch 90°- und 70°-Endoskope oder Zoomendoskope. Sobald Kamerakopf 32, Lichtleiter 36 und Endoskop 28 miteinander verbunden sind, kann eine erste digitale Video-Laryngoskopie durchgeführt werden, d.h. eine Aufnahme des Kehlkopfes. Die Aufzeichnung erfolgt gemäß dem ersten Betriebsmodus mit einer Auflösung von vorliegend exemplarischen 512 x 386 Bildpunkten in Farbe für Einzelbilder und einer Aufnahmegeschwindigkeit von 25-30 Bildern/Sekunde für Bildsequenzen. Das Pixelarray 36 gibt in diesem langsamen Vollbildmodus die Daten über den Ausgang 44 über eine Datenleitung direkt an das Steuergerät 16 in den digitalen Bildspeicher 17 aus, das die Daten wiederum - eventuell nach geeigneter Umkonvertierung in einen der Standardformate PAL bzw. NTSC - an den Video-Monitor 14 ausgibt. Der Arzt kann sich also während der Untersuchung den Kehlkopf über den Monitor 14 betrachten.

Über das Bedienelement 38 an der Kamera 12 kann dann der Arzt eine digitale Hochgeschwindigkeitsaufnahme durchführen, z.B. vorliegend exemplarisch mit einer Auflösung von 256 x 256 Bildpunkten in Farbe oder Schwarz-Weiß bei einer Aufnahmegeschwindigkeit von exemplarisch 4000 Bildern/Sekunde. Da das Bedienelement 38 an der Kamera 12 angeordnet ist, kann der Arzt das Umschalten ohne einen weiteren Handgriff durchführen und läuft somit insbesondere nicht Gefahr, die Kamera oder das Endoskop in ihrer Ausrichtung zu ändern, während er die Modusumstellung durchführt. Das Bedienelement ist vorzugsweise in einem Griffbereich 50 der Kamera angeordnet, so dass die bedienende Person, d.h. der Arzt, das Umschalten ohne Umgreifen und ohne Einsatz der zweiten Hand durchführen kann.

Nach dem Umschalten gibt das Pixelarray 36 die Daten des Feldes 40 an dem Ausgang 46 über die Datenleitung an das Steuergerät 16 aus. Dort werden sie im digitalen Bildspeicher 17 abgelegt. Es ist möglich, dass das Steuergerät 16 auch eine Umwandlung der Bilddaten in ein geeignetes Videoformat durchführt, um die Bildsequenz entweder als Zeitlupe oder in Echtzeit durch Auslassen einiger Bilder auf dem Monitor 14 mit der Monitorwiedergabefrequenz darzustellen.

Aufgrund der Betrachtung der Hochgeschwindigkeitsglottographie kann der Arzt sich dann entscheiden, eine digitale Video-Kymographie durchzuführen, indem er wiederum das Bedienelement 38 geeignet betätigt. In diesem Bildaufnahmemodus nimmt die Kamera 12 Bilder mit einer Auflösung von 512 x 1 Bildpunkten auf, d.h. pro zeitliche Abtasteinheit wird eine Zeile von Bildpunkten ausgelesen. Die Aufnahme erfolgt in Farbe oder Schwarz/Weiß bei einer Aufnahmegeschwindigkeit von vorliegend exemplarisch 8000-10000 Bildern/Sekunde oder höher. Über den Ausgang 48 gibt das Pixelarray 36 die Bilddaten der Bildzeile 42 über die Datenleitung an das Steuergerät 16 aus. Dort werden sie im digitalen Bildspeicher 17 abgelegt.

Alle im Bildspeicher 17 gelagerten Daten, die bei einer Aufnahme durch einen der drei Aufnahmemodi (Laryngoskopie, Hochgeschwindigkeitsaufnahme bzw. Kymograpie) entstanden sind, können vom Steuergerät 16 über die Verbindung 20 an den Computer 22 zur Archivierung weitergeleitet werden. Auf diesem Computer 22 sorgt eine geeignete Software (nicht abgebildet) für eine geeignete Anzeige und Auswertung der archivierten Bilddaten. An dem Computer 22 kann der Arzt beispielsweise für die Hochgeschwindigkeitsbildsequenzen über das Programm eine Schnittlinie über ein Standbild aus der Sequenz legen, die quer zu den Stimmlippen verläuft, um sich ein digitales Kymogramm entlang der entsprechenden Pixellinie er- und darstellen zu lassen.

Die im einzeiligen Modus ausgenommenen Kymographiebilder oder die durch das Programm erstellten digitalen Kymogrammdaten aus den Hochgeschwindigkeitsaufnahmen, d.h. jeweils die aufeinander folgenden Bildzeilen, können auf dem Computer dargestellt und analysiert werden.

Bevorzugterweise arbeitet die Kamera 12 derart, dass die Video- und Hochgeschwindigkeitsaufnahmen in den beiden flächenhaft aufnehmenden Bildaufnahmemodi in einem sogenannten "progressive scan"-Modus erzeugt werden, d.h. auf eine Weise, bei der alle Bildpunkte eines Frames zu einem Zeitpunkt gleichzeitig aufgenommen werden, und nicht, wie aus der Fernsehtechnik bekannt, in einem "Interlacing"-Modus mit zeilenweiser Verschränkung zweier zeitlich nacheinander aufgenommenen Halbbilder. Auf diese Weise werden durch Interlacing entstehende Bewegungsartefakte vermieden.

Der Arzt hat ferner die Möglichkeit, Datenströme, wie z.B. Messdaten oder dergleichen, von angeschlossenen externen Geräten 24 und 26 synchronisiert und simultan zu den Bildaufnahmen aufnehmen und archivieren zu lassen. Hierzu umfasst das Steuergerät 16 eine Synchronisationseinrichtung, die die Datenströme der externen Geräte 24, 26, wie z.B. das akustische Signal bzw. Audiosignal des Mikrophons 24 oder das Elektroglottogramm (EGG), mit den Bildern der Bildaufnahme synchronisiert. Weitere externe Geräte könnten beispielsweise ein Geschwindigkeitssensor sein. Ein weiteres exemplarisches extern zugeführtes Signal könnte beispielsweise ein Datenstrom von seriellen Schallpegelwerten sein.

Über den PC 22, für den keine Sonderhardware erforderlich ist, findet die Übertragung, Abspeicherung und Archivierung der Videosequenzen der Video-Laryngoskopie, die Hochgeschwindigkeitsaufnahmen der Hochgeschwindigkeitsglottographie und der Videokymogramme der Videokymographie sowie zudem wahlweise einzelner Standbilder statt.

Innerhalb des PC 22 lassen sich die aufgenommenen Daten, d.h. die Bilddaten und gegebenenfalls die Ton- bzw. E-lektroglottogrammdaten, mit einem Verfahren der digitalen Bild- und Signalverarbeitung auswerten, das beispielsweise in einer geeigneten Software implementiert ist, das auf dem PC läuft. Aus den Hochgeschwindigkeitsaufnahmen des schnelleren flächenhaft aufnehmenden Bildaufnahmemodus lassen sich beispielsweise deskriptive Parameter zur Beschreibung der Stimmlippenschwingungen berechnen, wie z.B. ein Öffnungs- und Verschlussquotient, Grundfrequenz rechts und links, Einschwing- und Ausschwingzeit rechts und links usw. Diese Werte lassen sich dann unter anderem mit Werten aus der Audio-Aufzeichnung und der EGG-Aufzeichnung korrelieren. Aus den hochaufgelösten Farblaryngoskopieaufnahmen, d.h. den Aufnahmen in dem langsameren Vollbildmodus, lassen sich mit Hilfe von Farbtexturverfahren beispielsweise Diagnosenvorschläge für die Inzidenzleukoplakie als Krebsvorstufe berechnen.

Das im vorhergehenden beschriebene System übertrifft folglich alle derzeit üblichen Systeme. In der derzeitigen klinischen Praxis wird die Morphologie, die Textur und die Farbe des Kehlkopfs durch Video-Laryngoskopie diagnostiziert, die Analyse der Grob- und Feinbewegungen der Stimmlippen wird dagegen durch die sub-optimale Video-Stroboskopie durchgeführt. In einigen europäischen, japanischen und US-Amerikanischen Kliniken und Zentren erfolgt zudem für eine Differenzialdiagnose der Stimmlippenschwingungen eine zusätzliche zeitaufwändige Aufnahme mit einem weiteren System, entweder mit einer digitalen Hochgeschwindigkeitskamera oder mit einem Video-Kymographie-System. Das im vorhergehenden beschriebene Ausführungsbeispiel eines Gerätes zur Kehlkopf- und Stimmbänderuntersuchung schafft dagegen eine kombinierte Multifunktionskameravorrichtung zur objektivierten Stimmdiagnose und, genauer, die Integration eines umschaltbaren digitalen (farbigen) Video-Laryngoskopie-Systems mit einer digitalen (Farb-)Hochgeschwindigkeitskamera, wobei auch die Aufnahme von digitalen (Farb-)Videokymogrammen ermöglicht wird.

Bezugnehmend auf die vorhergehende Beschreibung wird noch auf folgendes hingewiesen. Obwohl im vorhergehenden beschrieben worden ist, dass die Bedieneinheit am Kamerakopf selbst angebracht ist, kann es ferner vorgesehen sein, dass das Umschalten der Kamera zwischen den einzelnen Betriebsmodi über beispielsweise die Tastatur (nicht gezeigt) des PCs 22 oder über das Mikrophon 24 oder einen Fußschalter (nicht gezeigt) durchgeführt wird. Ferner können die Sensorfläche für die Laryngoskopie 36, die Sensorzeile für die digitale Kymographie 42 und die Sensorfläche 40 für die Hochgeschwindigkeitsaufnahmen auch anders angeordnet sein. Ferner wird noch darauf hingewiesen, das es bei Ankopplung einer Stroboskoplichtquelle freilich auch möglich wäre, mit oben beschriebenem gerät eine Video-Stroboskopie durchzuführen.

Es wird darauf hingewiesen, dass die oben beschriebene Kamera nicht nur zur Untersuchung von Stimmlippen geeignet ist, sondern allgemein zur Untersuchung von sich mit hoher Frequenz bewegenden Objekten, wie z.B. neben Stimmlippen auch zur Untersuchung von künstlichen oder natürlichen Herzklappen, oder zur zeitlich aufgelösten Aufnahme von Stimmersatzgebung nach Kehlkopfentfernung.

Ferner wird darauf hingewiesen, dass obiges Ausführungsbeispiel darin lediglich ein bevorzugtes Ausführungsbeispiel betraf, dass eine Kamera mit drei Betriebsmodi beschrieben wurde. Die Kamera bietet aber auch schon dann Vorteile gegenüber früheren Systemen, wenn lediglich zwei der drei oben beschriebenen Modi implementiert sind.

Bezugnehmend auf die vorhergehende Beschreibung wird noch darauf hingewiesen, dass das System nach Fig. 1 auch mit zwei unterschiedlichen Kameraköpfen, nämlich einem farbfähigen mit einer Farbkamera und einem nicht-farbfähigen mit einer lichtstärkeren Schwarz-Weiß-Kamera, ausgestattet sein könnte. Wahlweise könnte einer der beiden Kameraköpfe mit dem Steuergerät verbunden werden. Beide Kameraköpfe wären vorzugsweise mit wahlweise einem von zwei Endoskopen verbindbar, die in dem System enthalten wären, nämlich einem Stabendoskop und einem Fiberskop. Jedoch wäre es ferner möglich, dass der nicht-farbfähige aber dafür lichtempfindlichere Kamerakopf fest mit dem Fiberskop versehen wäre, während der andere farbfähige Kamerakopf mit dem Stabendoskop versehen wäre. Das Vorsehen einer solchen Ausrüstung für das System von Fig. 1 hätte den Vorteil, dass bei Benutzung des Fiberskops die dadurch bedingte geringe Ausleuchtung durch die höhere Lichtempfindlichkeit des Schwarz-Weiß-Kamerakopfes unter Verzicht auf die Farbinformation ausgeglichen werden kann, während bei Benutzung des Stabendoskops die vergleichsweise höhere Ausleuchtung dazu verwendet werden kann, den farbfähigen Kamerakopf einsetzen zu können.

Ferner wird bezugnehmend auf die vorhergehende Beschreibung noch darauf hingewiesen, dass zwar in vorhergehender Beschreibung stets zwischen Kamerakopf einerseits und Endoskopaufsatz andererseits unterschieden worden ist, dass diese Unterscheidung aber überflüssig werden kann, wenn der im vorhergehenden erwähnte CMOS- oder CCD-Sensor beispielsweise direkt in das Endoskop integriert wird, um eine Bildaufnahmevorrichtung zu ergeben, die fest mit dem Endoskop gekoppelt ist. Hierbei kann der Sensor-Chip am Endoskop sowohl distal, d.h. am vorderen, dem Kehlkopf zugewandten Ende des Endoskops bzw. an der Endoskopspitze gelegen, als auch proximal, d.h. an dem dem Arzt zugewandten Ende des Endoskops gelegen, angeordnet sein. Der im vorhergehenden erwähnte Kamerakopf reduzierte sich hierbei zu lediglich einem Griff einer so entstehenden Bildaufnahmevorrichtung, in der das lichtempfindliche Pixelarray in das Endoskop integriert ist.

Schließlich wird bezugnehmend auf Fig. 1 noch darauf hingewiesen, dass der Speicher 17 auch im Kamerakopf angeordnet sein könnte.

## Patentansprüche

1. Bildaufnahmevorrichtung mit
einem Pixelarray (36) von regelmäßig in Zeilen und Spalten angeordneten Bildpunkten, wobei das Pixelarray (36) ein Farbpixelarray (36) ist;
einem Endoskop (28) zur Abbildung von Stimmlippen oder anderer sich mit hoher Frequenz bewegender Objekte wie beispielsweise Herzklappen auf das Pixelarray (36);
einem ersten Bildaufnahmemodus zur Bildaufnahme mit einer ersten Auflösung, die eine erste Anzahl von Bildpunkten aufweist, mit einer ersten Bildwiederholfrequenz;
einem zweiten Bildaufnahmemodus zur Bildaufnahme mit einer zweiten Auflösung, die eine zweite Anzahl von Bildpunkten aufweist, mit einer zweiten Bildwiederholfrequenz, wobei die erste Anzahl größer als die zweite Anzahl und die erste Bildwiederholfrequenz kleiner als die zweite Bildwiederholfrequenz ist,
wobei die Bildaufnahmevorrichtung zwischen den Bildaufnahmemodi umschaltbar ist, wobei
der erste Bildaufnahmemodus derart ausgebildet ist, dass die Bildaufnahme mit der ersten Auflösung eine Aufzeichnung der Bildpunkte innerhalb eines ersten rechteckigen Bereiches (36) des Pixelarrays in Farbe aufweist, um ein zweidimensionales Feld der ersten Anzahl von Bildpunkten zu umfassen, und der zweite Bildaufnahmemodus derart ausgebildet ist, dass die Bildaufnahme mit der zweiten Auflösung eine Aufzeichnung der Bildpunkte innerhalb eines zweiten rechteckigen Bereiches (40) des Pixelarrays aufweist, um ein zweidimensionales Feld der zweiten Anzahl von Bildpunkten aufzuweisen, und wobei eine Ausdehnung des ersten rechtekkigen Bereiches (36) größer als die Ausdehnung des zweiten rechteckigen Bereiches (40) ist.

2. Bildaufnahmevorrichtung gemäß Anspruch 1, bei der die erste Bildwiederholfrequenz zwischen 20 und 1000 Hz liegt, und die zweite Bildwiederholfrequenz größer als 1000 Hz ist.

3. Bildaufnahmevorrichtung gemäß Anspruch 1 oder 2, bei der die erste Anzahl etwa 350 x 350 bis 1000 x 1000 und die zweite Anzahl etwa 200 x 200 bis 500 x 500 beträgt.

4. Bildaufnahmevorrichtung gemäß Anspruch 1, bei dem die Bildaufnahmevorrichtung ferner folgendes Merkmal aufweist:
einen dritten Bildaufnahmemodus zur Bildaufnahme mit einer dritten Auflösung, die ein eindimensionales Feld (42) einer dritten Anzahl von Bildpunkten aufweist, mit einer dritten Bildwiederholfrequenz, wobei die dritte Anzahl kleiner als die erste Anzahl und die dritte Bildwiederholfrequenz größer als die erste Bildwiederholfrequenz ist,
wobei die Bildaufnahmevorrichtung zwischen den drei Betriebsmodi umschaltbar ist.

5. Bildaufnahmevorrichtung gemäß Anspruch 4, bei der die erste Bildwiederholfrequenz zwischen 20 und 1000 Hz liegt, die zweite Bildwiederholfrequenz zwischen 1000 und 4000 Hz, und die dritte Bildwiederholfrequenz größer als 4000 Hz ist.

6. Bildaufnahmevorrichtung gemäß Anspruch 4 oder 5, bei der die erste Anzahl etwa 350 x 350 bis 1000 x 1000, die zweite Anzahl etwa 200 x 200 bis 500 x 500 und die dritte Anzahl 350 x 1 bis 1000 x 1 beträgt.

7. Bildaufnahmevorrichtung gemäß einer der Ansprüche 4-6, bei der die dritte Bildwiederholfrequenz größer ist als die zweite Bildwiederholfrequenz.

8. Bildaufnahmevorrichtung gemäß einem der vorhergehenden Ansprüche, bei der das zweidimensionale Feld der ersten und der zweiten Anzahl in etwa zentrisch zu einer optischen Achse der Bildaufnahmevorrichtung angeordnet sind.

9. Bildaufnahmevorrichtung gemäß einer der vorhergehenden Ansprüche, die zur zeitlich aufgelösten Aufnahme von schwingenden Stimmlippen, von natürlichen oder künstlichen Herzklappen oder zur zeitlich aufgelösten Aufnahme von Stimmersatzgebung nach Kehlkopfentfernung geeignet ist.

10. Bildaufnahmevorrichtung gemäß einer der vorhergehenden Ansprüche, die ferner folgendes Merkmal aufweist:
ein Bedienelement (38) zum Umschalten der Bildaufnahmevorrichtung zwischen den Bildaufnahmemodi.

11. Gerät zur Untersuchung eines Kehlkopfes und von schwingenden Stimmlippen, mit
einer Lichtquelle (18);
einer Bildaufnahmevorrichtung (12) gemäß einem der vorhergehenden Ansprüche, wobei die Bildaufnahmevorrichtung (12) ein Endoskop (28) zum Leiten eines Lichtes von der Lichtquelle (18) auf die Stimmlippen zur Beleuchtung und zum Abbilden reflektierten Lichtes auf das Pixelarray (36) aufweist.

12. Gerät gemäß Anspruch 11, mit
einer Steuereinrichtung (16), die mit der Bildaufnahmevorrichtung (12) gekoppelt und mit einem externen Gerät (24, 26) koppelbar ist, wobei die Steuereinrichtung ferner folgende Merkmale aufweist:
eine Einrichtung (16) zum Synchronisieren eines Datenstroms von dem externen Gerät (24, 26) mit der Bildaufnahme.

13. Gerät gemäß Anspruch 13 oder 12, mit
einem Bildspeicher (17) zur Zwischenspeicherung der Bilddaten zwischen dem Aufnehmen und dem Archivieren.

14. Gerät gemäß Anspruch 13, bei dem der Bildspeicher (17) extern zu der Bildaufnahmevorrichtung in einer Steuereinrichtung angeordnet ist, die mit der Bildaufnahmevorrichtung (12) gekoppelt ist.

15. Gerät gemäß einem der Ansprüche 11 bis 14, bei dem die Bildaufnahmevorrichtung eine Bildaufnahmevorrichtung gemäß einem der Ansprüche 4 bis 14 ist, und das ferner folgendes Merkmal aufweist:
eine Steuereinrichtung, die mit der Bildaufnahmevorrichtung (12) gekoppelt ist, zum Anzeigen eines Kymogramms aus dem Bildaufnahmemodus mit einer Auflösung, die das eindimensionale Feld (42) aufweist.

16. Gerät gemäß einem der Ansprüche 11 bis 14, bei dem die Bildaufnahmevorrichtung eine Bildaufnahmevorrichtung gemäß einem der Ansprüche 4-7 ist, und das ferner folgendes Merkmal aufweist:
eine Steuereinrichtung, die mit der Bildaufnahmevorrichtung (12) gekoppelt ist, zum Ermöglichen, dass ein Benutzer in einem Standbild aus dem zweiten Bildaufnahmemodus eine Bildpunktlinie aus dem zweidimensionalen Feld (40) der zweiten Anzahl bestimmt, und zur Erzeugung und Anzeige eines Kymogramms aus Bilddaten des dritten Bildaufnahmemodus bezüglich der angezeigten Bildpunktlinie.

17. Gerät gemäß einem der Ansprüche 11 bis 16, bei dem der erste Bildaufnahmemodus der Laryngoskopie, der zweite Bildaufnahmemodus zur Hochgeschwindigkeitsbildaufnahme und der dritte Bildaufnahmemodus der Kymographie dient.

18. Gerät gemäß einem der Ansprüche 11 bis 17, bei dem das Pixelarray der Bildaufnahmevorrichtung entweder distal oder proximal im Endoskop angeordnet ist.

19. System zur Untersuchung eines Kehlkopfes und von schwingenden Stimmlippen, mit
einer Lichtquelle (18);
einer ersten Bildaufnahmevorrichtung gemäß einem der Ansprüche 1 bis 10, wobei die erste Bildaufnahmevorrichtung farbfähig ist;
einer zweiten Bildaufnahmevorrichtung gemäß einem der Ansprüche 1 bis 10, wobei die zweite Bildaufnahmevorrichtung nicht-farbfähig aber dafür lichtempfindlicher als die erste ist; und
zumindest einem Endoskop (28) zum Leiten eines Lichtes von der Lichtquelle (18) auf die Stimmlippen zur Beleuchtung und zum Abbilden reflektierten Lichtes auf das Pixelarray (36) der ersten oder zweiten Bildaufnahmevorrichtung.

20. System gemäß Anspruch 19, bei dem das Endoskop wahlweise mit der ersten oder der zweiten Bildaufnahmevorrichtung wirksam koppelbar ist.

21. System gemäß Anspruch 19, bei dem das Endoskop ein starres Endoskop ist, und das ferner ein Fiberskop aufweist, wobei beide wahlweise mit der ersten oder der zweiten Bildaufnahmevorrichtung wirksam koppelbar sind.

22. System gemäß Anspruch 19, bei dem das Endoskop ein starres Endoskop ist, und das ferner ein Fiberskop aufweist, wobei das starre Endoskop fest mit der ersten Bildaufnahmevorrichtung wirksam gekoppelt ist, während das Fiberskop fest mit der zweiten Bildaufnahmevorrichtung wirksam gekoppelt ist.
